# EUROPEAN PATENT APPLICATION

(11) **EP 1 184 458 A1**
(43) Date of publication of application: **06.03.2002**
(21) Application number: 00202991.6
(22) Date of filing: 28.08.2000
(51) Int. Cl.: C12N 15/13, C07K 16/28, C07K 19/00, A61K 39/395, A61K 47/48, A61P 35/00, G01N 33/577, G01N 33/68, C12N 5/04, C12N 5/06, C12N 5/08, C12N 5/10, A01K 67/027, C12N 15/85

(54) **Differentially expressed CD46 epitopes, proteinaceous molecules capable of binding thereto, and uses thereof**

(71) Applicant: U-BiSys B.V., 3584 CX Utrecht (NL)
(72) Inventor: LOGTENBERG, Ton, 3433-CH / Nieuwegein (NL); CILENTI, Lucia, 82020 Foicino di Valfortone (BN) (IT); BLOEM, Andries Christiaan, 3971-BL / Driebergen (NL); ZWIJSEN, Renate Marie Louise, 3583-VE / Utrecht (NL)
(74) Representative: Prins, Adrianus Willem

(57) **Abstract**

Provided are among others epitopes, methods for finding these epitopes and proteinaceous molecules capable of binding to said epitopes.

In one aspect the invention provides a proteinaceous molecule capable of specifically binding to an epitope present in a subset of CD46 proteins. Said molecule is capable of distinguishing between CD46 proteins belonging to the subset and CD46 proteins not belonging to the subset. The proteinaceous molecule is preferably an antibody.
Medicaments and uses of proteinaceous molecules are provided .

## Description

The invention relates to the field of medicine. The invention in particular relates to the diagnostic and/or medical use of binding molecules.

B-lymphocytes can produce antibodies in response to exposure to biological substances like bacteria, viruses and their toxic products. Antibodies are generally epitope specific and bind strongly to said biological substances carrying these epitopes. The hybridoma technique (Köhler and Milstein 1975) makes use of the ability of the B cells to produce monoclonal antibodies to specific antigens and to subsequently isolate and produce monoclonal antibodies by fusing B cells from mice exposed to the antigen of interest to immortalized murine plasma cells. This technology resulted in the realization that monoclonal antibodies produced by hybridoma's could be used in research, diagnostics and therapies to treat different kinds of diseases like cancer and auto-immune related disorders.

Because antibodies that were produced in mouse hybridoma's induced dramatic immune responses in humans, it became clear that the antibodies that were required for successful treatments of diseases in human needed to fit the human requirements to lower these immune responses. For this, murine antibodies were first engineered by replacing the murine constant domains with human constant regions. Subsequently, domains between the variable domains, that specify the antigen binding were replaced by their human counterparts. The final stage in this humanization process is the production of fully human antibodies. To date many different diseases are being treated with either humanized or fully human antibodies. Nevertheless, many disorders are not being treated since no specific epitopes are found, that are expressed on cells or substances that need to be removed by the immune system through the interaction with antibodies or other proteinaceous molecules capable of binding said epitope.

The present invention provides among others novel epitopes, methods for finding these epitopes and proteinaceous molecules capable of binding to said novel epitopes.

In one aspect the invention provides a proteinaceous molecule capable of specifically binding to an epitope present in a subset of CD46 proteins. Said molecule is capable of distinguishing between CD46 proteins belonging to the subset and CD46 proteins not belonging to the subset. The proteinaceous molecule can thus be used to type samples containing CD46 protein. This property is useful in, for instance, CD46 purification strategies.

CD46 is a protein that is widely expressed on many different cell types and tissues. In a preferred embodiment a proteinaceous molecule of the invention is capable of essentially specifically binding to a subset of CD46 expressing cells. A cell belonging to said subset of cells comprises a detectable amount of CD46 protein comprising said epitope. This cell can also comprise CD46 protein not comprising said epitope. A CD46 positive cell that does not belong to the subset contains CD46 protein not comprising said epitope. Typically this cell does not comprise detectable levels of CD46 protein comprising said epitope, however, this may not always be true. With essentially specifically binding to a subset of CD46 positive cells is meant that the number of CD46 proteins comprising said epitope on a cell not belonging to said subset, is too low to be detected or to exert a biological effect upon binding of a proteinaceous molecule of the invention.

Preferably a cell belonging to said subset of cells comprises a neoplastic cell. It has been found that many CD46 proteins of neoplastic cells comprise at least one epitope that is essentially not present on CD46 proteins expressed on normal CD46 positive cell types. Preferably, said neoplastic cell is derived from a hemopoietic cell, a cervix cell, a colon cell, a kidney cell or a liver cell. In a particularly preferred embodiment said neoplastic cell is derived from a B-cell. More preferably said neoplastic cell comprises a Multiple Myeloma (MM) cell.

An epitope can consist of any (combination of) substance(s). Typically an epitope is formed by an amino acid sequence, a sugar or lipid moiety, a part post translationally modified or a combination of these elements. An epitope presents substance(s) making up the epitope in a form that can bind a binding molecule. When the epitope is not present, one or more of the substances making up the epitope are not available for binding to a binding molecule. Said one more substances making up the epitope do not have to be absent from the molecule. All said substance(s) can still be present in or on the CD46 protein, however in this case, said one or more substances are present in a form that does not allow binding of a proteinaceous molecule of the invention. Typically, this is the case when the binding molecule is prevented from binding due to sterical hindrance and/or due to a conformational change in the CD46 protein leading to a different distribution of said one or more of the substance(s) in said protein. In one aspect the invention provides different glycosylation forms of CD46. At least one variant glycosylation form of CD46 comprises an epitope that is expressed on e.g. MM cells, whereas said epitope is not expressed by normal CD46 positive cell types. A similar epitope is present in many other neoplastic cells (described below). The CD46 epitope present on MM cells thus is a suitable marker for at least some types of tumor cells. The invention therefore provides the use of a proteinaceous molecule of the invention for the typing of a CD46 positive cell. Preferably, said use comprises a diagnostic use.

To date, CD46 proteins of various animal species have been isolated. A person skilled in the art is well capable of identify CD46 protein in species from which the CD46 protein is not yet determined. A suitable strategy is to use the information from an identified CD46 in an evolutionary closely related species. This can be the nucleic acid and/or amino acid sequence (for homology hybridization of nucleic acid libraries under more or less stringent conditions or nucleic acid amplification strategies using primers for conserved evolutionary conserved regions). Alternatively, antibodies specific for conserved parts of a CD46 molecule of an evolutionary related species can be used to identify a CD46 protein in another species. In a preferred embodiment said CD46 comprises a mammalian CD46 protein. More preferably, said CD46 protein comprises a human CD46 protein.

A proteinaceous molecule of the invention can be any type of binding molecule known in the art. A binding molecule is capable of specifically binding an epitope, meaning that the binding molecule does not bind efficiently to proteins not comprising said epitope. Many different binding molecules are known in the art. In a preferred embodiment a proteinaceous molecule of the invention comprises an antibody or a functional part or derivative thereof. An antibody may be produced or first generated by a B-cell. However, currently, many different ways of producing artificial antibodies are known in the art. An artificial antibody comprises a similar structure as a classical antibody. Such artificial antibodies can for instance be generated by in vitro assembly of amplified nucleic acid coding for various parts of an antibody. A functional part of an antibody comprises at least an part involved in epitope binding. A functional part of an antibody typically comprises the same epitope binding capacity in kind not necessarily in the amount. A person skilled in the art is well capable of altering parts of the amino-acid sequence of an antibody without essentially affecting the binding capacity of said antibody. Alterations can comprise deletions, insertions, amino-acid substitutions or a combination of these alterations. Such derivatives of antibodies are also part of the invention.

Much interest is currently directed toward the use of binding molecules in the treatment of human disease. One application is the use of binding molecules to facilitate removal of undesired cells from a body. In this application the binding molecule must comprise sufficient specificity for the cells to be removed in order not to result in undesired side effects. Typically, but not necessarily such binding molecules comprise antibodies. An antibody is capable of binding to an epitope expressed by an undesired cell and thereby mark said cell for removal from the body. This can be done in several ways for instance mediated by the immune system or the complement system or a combination thereof. Removal can also be achieved in (combination with) other ways.

In the art it has been observed that antibodies comprising a substantial amount of murine sequences do not perform very well humans. Typically, the pharmacodynamics of such molecules are not comparable to the dynamics of a fully human or humanized antibody. Another disadvantage is that murine protein sequences are capable of eliciting a strong immune response in humans thereby further decreasing the utility of such murine sequence containing antibodies in humans. Preferably, an antibody of the invention is a human antibody or a humanized antibody. Such antibodies closely resemble normal human antibodies and have similar pharmacodynamics upon administration to humans.

In a preferred embodiment a proteinaceous molecule of the invention comprises a tag. A tag can be used for detection purposes. Alternatively, a tag can comprise a toxic substance. A toxic substance can enhance removal of targeted cells from the body. In one embodiment said toxic substance comprises a toxin and/or a radioactive substance.

In another embodiment the invention provides a method for the treatment of an individual suffering from or at risk of suffering from a disease, comprising administering to said individual a therapeutically acceptable amount of a proteinaceous molecule of the invention. Administration can be used to facilitate removal of, undesired cells that cause at least part of the disease from the body of said individual. Such cells may be present in said body upon administration or said individual may be at risk of comprising said undesired cells. In a preferred embodiment said disease is a neoplastic disease. The invention also provides the use of a proteinaceous molecule of the invention for the preparation of a medicament. Preferably, said medicament is used for the treatment of neoplastic disease. A proteinaceous molecule of the invention may be used in conjunction with other methods of treatments or medicaments. In a preferred embodiment, said other treatment or medicament comprises another antibody comprising a specificity for another epitope. Preferably, said another epitope is present on a CD46 expressing cell. Since CD46 is involved in the complement pathway, a molecule of the invention can be used to modulate complement mediated effects of said antibody specific for said another epitope. Modulation can comprise stimulation or inhibition of complement activity toward cells capable of binding both, a proteinaceous molecule of the invention and said antibody specific for said another epitope.

In one aspect of the invention, a proteinaceous molecule of the invention is used to type a cell. Now that different forms of CD46 can be detected it is possible to use this property in detection methods. In a non-limiting example a proteinaceous molecule of the invention is used to determine whether cells in sample comprise neoplastic cells. Preferably, said neoplastic cell comprises a multiple myeloma cell. In another aspect the invention provides the use of an epitope expressed on a subset of CD46 expressing cells as a marker for tumor cells. The invention therefore further provides a kit comprising at least a proteinaceous molecule of the invention. Said kit preferably, further comprises a buffer suitable for allowing specific binding.

In yet another aspect the invention provides a nucleic acid encoding a proteinaceous molecule according to the invention, or a part involved in CD46 binding of such a molecule. Such nucleic acid can be obtained in various ways. One non-limiting example is amplification of nucleic acid encoding said proteinaceous molecule from a cell expressing said molecule. In many methods for the generation of binding molecules and particularly for artificial antibodies, nucleic acid coding for said binding molecule is readily available.

A proteinaceous molecule of the invention can be produced in variety of ways. In a preferred embodiment a molecule of the invention is produced by a cell comprising a nucleic acid encoding said proteinaceous molecule. Preferably, said cell is primate, rodent, bird or plant cell. Preferably, said cell is human cell. Human cells and the closely related primate cells have very similar post-translational modification machineries. In this way a proteinaceous molecule of the invention can be provided with human-like and more preferably, human post-translational modifications such as glycosylation. Such human type modification is less immunogenic in humans than modifications of other by cells of other species, thus leading to a better efficacy of the treatment. In a preferred embodiment said cell further comprises a means for the conditional expression of a nucleic acid of interest. In this way, expression of a proteinaceous molecule of the invention can be induced at times when expression is desired. This is advantageous when expression of said proteinaceous molecule interferes with a function of said cell. By essentially avoiding expression during normal culture of the cells, the cells can be cultured and expanded normally before the condition for expression of the nucleic acid is fulfilled. A preferred system for the conditional expression of nucleic acid of interest comprises a tetracycline responsive expression system. The invention therefore also provides a cell comprising a nucleic acid encoding a proteinaceous molecule of the invention , said cell further comprising a tetracycline responsive molecule capable of influencing expression of a promoter.

In a preferred embodiment said cell further comprises nucleic acid encoding an early protein of adenovirus or a functional part, derivative and/or analogue thereof. Such a cell is capable of producing more functional proteinaceous molecule per time unit. In a preferred embodiment said early protein comprises adenovirus E1 or a functional part, derivative and/or analogue thereof. Adenovirus E1 comprises transcriptional activation properties and generally has the effect of enhancing protein production in a cell. In a preferred embodiment said adenovirus early protein comprises adenovirus E2A or a functional part, derivative and/or analogue thereof. E2A in a cell has a protein production enhancing effect. Derivatives of E1 or E2A can be generated in various ways. One non-limiting way is through amino-acid substitution. A functional part and/or derivative of adenovirus E1 or E2A comprises the same protein production enhancing qualities in kind not necessarily in amount. Many viruses use proteins with similar protein production enhancing qualities as adenovirus E1 or E2A. Such molecules form suitable analogues of E1 or E2A.

In another aspect the invention provides a plant or non-human animal comprising a cell capable of producing a proteinaceous molecule of the invention. Typically, though not necessarily such a plant or non-human animal is transgenic for a nucleic acid encoding a proteinaceous molecule of the invention. In one embodiment said animal comprises a human immunoglobulin locus or a functional part thereof.

In yet another aspect the invention provides a gene delivery vehicle comprising a proteinaceous molecule according of the invention. Such a gene delivery vehicle can be used to target delivery of nucleic acid contained in said gene delivery vehicle to cells expressing a CD46 protein belonging to the subset of CD46 proteins.

In yet another aspect the invention provides a method for a selecting a proteinaceous molecule capable of binding specifically to an epitope on a protein wherein said epitope is present on a subset of cells expressing said protein, said method comprising providing a collection of cells comprising cells of one type, with a library of proteinaceous binding molecules and selecting from proteinaceous molecules bound to said collection of cells at least one proteinaceous molecule capable of binding to said epitope.

### Detailed description

Antibodies can be produced by cells of the immune system, so-called B lymphocytes, in response to encounter with potentially harmful micro-organisms such as viruses, bacteria and their toxic products. Antibodies can be capable of binding strongly to the invading micro-organisms or their products that elicited their production and aid in their elimination. The activity of the immune system of producing antibodies in response to an invading micro-organism has been exploited in the production of monoclonal antibodies, a technology developed by Köhler and Milstein (1975). In the old definition, monoclonal antibodies are all those immunoglobulin molecules that are produced by the progeny of a single B lymphocyte. Conventionally, monoclonal antibodies are obtained by immunizing a mouse with an antigen and fusing the spleen or lymphnode B lymphocytes with an immortalized murine plasma cell line. The ensuing hybrid cell lines, or so-called hybridomas, bear the characteristics of both parental cell types: they are immortal and produce a single species of monoclonal antibody specific for the antigen used to immunize the mouse. The advantage of monoclonal antibodies is that they represent a homogeneous population of immunoglobulin molecules with a pre-defined binding specificity. Over the years, monoclonal antibodies have proven invaluable tools in research and diagnostics.

Soon after the invention of the hybridoma technology, the enormous potential of monoclonal antibodies in human therapy was realized. Because of their high binding specificity, antibodies were hypothesized, among others, to be capable of binding to viruses and bacteria and their toxic products facilitating their elimination. In other applications, monoclonal antibodies were envisaged to specifically bind to tumor cells to promote their eradication or to bind to soluble molecules produced by cells of the immune system to neutralize their activity in harmful chronic inflammatory conditions and/or in autoimmune disease. Indeed, monoclonal antibodies have been described as magic bullets that could be used in the treatment of a wide variety of human diseases (Bodey et al. 2000).

Numerous clinical studies with monoclonal antibodies of non-human, usually murine origin showed that they performed poorly as a result of their immunogenicity. Upon injection of murine monoclonal antibodies in humans, the human immune system recognizes the murine monoclonal antibodies as foreign proteins resulting in the induction of an immune response against the murine protein (Miller et al. 1983; Shawler et al. 1985). In addition, murine monoclonal antibodies have poor pharmacokinetic properties in humans (Riechmann et al. 1988) and are inefficient in recruiting effector functions of cells of the immune system (Hakini et al. 1991; Stephens et al. 1995). These issues have spurred the development of alternative strategies to obtain more human monoclonal antibodies for therapy (reviewed in Vaughan et al. 1998).

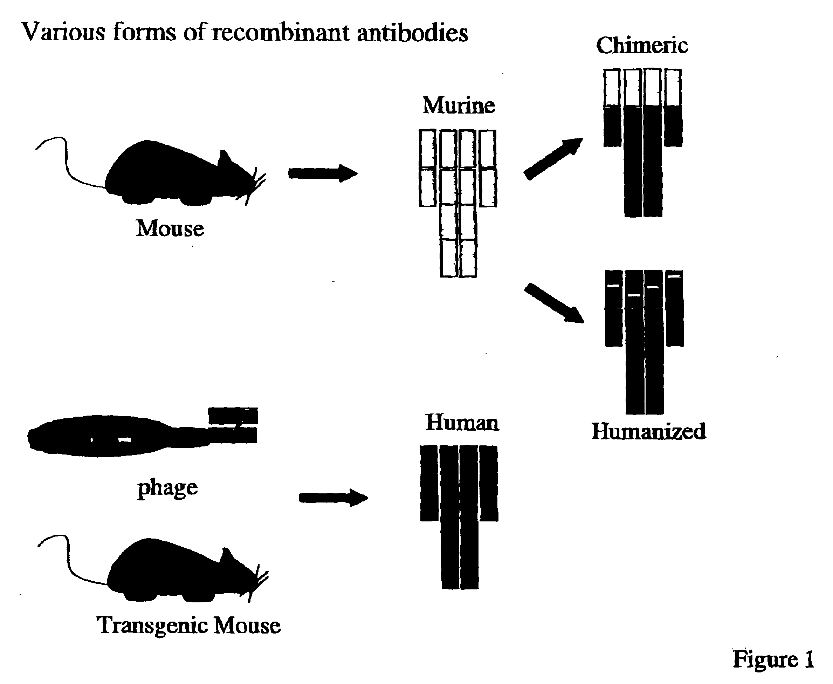

In one approach of creating more human monoclonal antibodies, the immunoglobulin variable regions of the murine monoclonal antibodies are genetically fused to human immunoglobulin constant regions (Fig. 1). The resulting chimeric monoclonal antibody still contains >30% murine amino acid sequences. Clinical application in humans of chimeric monoclonal antibodies has shown that these proteins retain their immunogenicity in the majority of cases (Khazadi et al. 1989; Elliot et al. 1994). In another approach, only immunoglobulin variable region sequences relevant for monoclonal antibody specificity are of murine origin; the constant regions of the immunoglobulin molecule as well as the framework regions of the variable region are of human origin (Fig.1). Clinical application of these 'humanized' monoclonal antibodies indicates that these molecules are generally more effective and have no or little intrinsic toxicity or immunogenicity (Jones et al. 1986). However, reconstructing the original affinity and specificity in a humanized version of a murine monoclonal antibody is a time-consuming process and may render the monoclonal antibody not enough human to completely prevent anti-antibody responses (Foote et al. 1992).

These considerations with chimeric and humanized monoclonal antibodies and the recent clinical success of engineered monoclonal antibodies spurred the development of efficient methods for the isolation and production of fully human monoclonal antibodies, the most desirable monoclonal antibody format for clinical application. The conventional methods to obtain murine and humanized monoclonal antibodies and two novel methods for obtaining monoclonal antibodies with complete human sequences are displayed in Fig.1.

One method of obtaining human monoclonal antibodies employs transgenic mice harboring human immunoglobulin loci in combination with conventional hybridoma technology (Bruggeman et al. 1996; Mendez et al. 1997). In these mice, large portions of human immunoglobulin heavy and light chain loci have been inserted in the mouse germ line while the endogenous murine immunoglobulin loci have been silenced by gene knockout. Immunization of these transgenic mice with an antigen results in the production of human antibodies specific for the antigen. Human monoclonal antibody-producing cell lines can be obtained from these mice by fusing the spleen cells of immunized mice with plasma cell lines in vitro to obtain immortalized monoclonal antibody-secreting hybridomas. Importantly, production of human monoclonal antibodies in transgenic mice depends on immunization procedures and is governed by constraints of the murine immune response. As a consequence, it is difficult if not impossible to obtain antibodies against the mouse's own antigens (autoantigens), to xenoantigens that have a high degree of homology to murine autoantigens or to antigens that have poor immunogenic properties such as polysaccharides. These notions have spurred the development of molecular approaches that obviate the need for immunization and cell 'immortalisation' to obtain human monoclonal antibodies with desired specificities. These strategies are based on immortalisation of the immunoglobulin genes encoding the monoclonal antibodies rather than the cell lines producing them.

Another method to obtain fully human monoclonal antibodies with desirable binding properties employs phage display libraries. This is an in vitro, recombinant DNA-based, approach that mimics key features of the humoral immune response (Winter et al. 1994; Burton et al. 1994). For the construction of phage display libraries, collections of human monoclonal antibody heavy and light chain variable region genes are expressed on the surface of bacteriophage particles, either in single chain Fv (scFv) or in Fab format. Large libraries of antibody fragment-expressing phages typically contain > 10⁹ antibody specificities and may be assembled from the immunoglobulin V regions expressed in the B lymphocytes of immunized or non-immunized individuals. Alternatively, phage display libraries may be constructed from immunoglobulin variable regions that have been partially assembled in vitro to introduce additional antibody diversity in the library (semi-synthetic libraries). For example, in vitro assembled variable regions contain stretches of synthetically produced, randomized or partially randomized DNA in those regions of the molecules that are important for antibody specificity.

Recombinant phages expressing antibody fragments of desirable specificities may be selected from a library by one of several methods. Target antigens are immobilized on a solid phase and subsequently exposed to a phage library to allow binding of phages expressing antibody fragments specific for the solid phase-bound antigen. Non-bound phages are removed by washing and bound phages eluted from the solid phase for infection of Escherichia coli bacteria and subsequent propagation. Multiple rounds of selection and propagation are usually required to sufficiently enrich for phages binding specifically to the target antigen. Phages may also be selected for binding to complex antigens such as complex mixtures of proteins or whole cells. Selection of antibodies on whole cells has the advantage that target antigens are presented in their native configuration, unperturbed by conformational changes that are introduced by immobilizing an antigen to a solid phase. The constraints imposed by the natural immune response and the influence of the immunogenicity of the target antigen do not permit the isolation of monoclonal antibodies against any antigen by conventional hybridoma technology. In phage approaches, these factors do not play a role, allowing the isolation of monoclonal antibodies directed against 'difficult' antigens such as autoantigens, carbohydrates and toxic antigens.

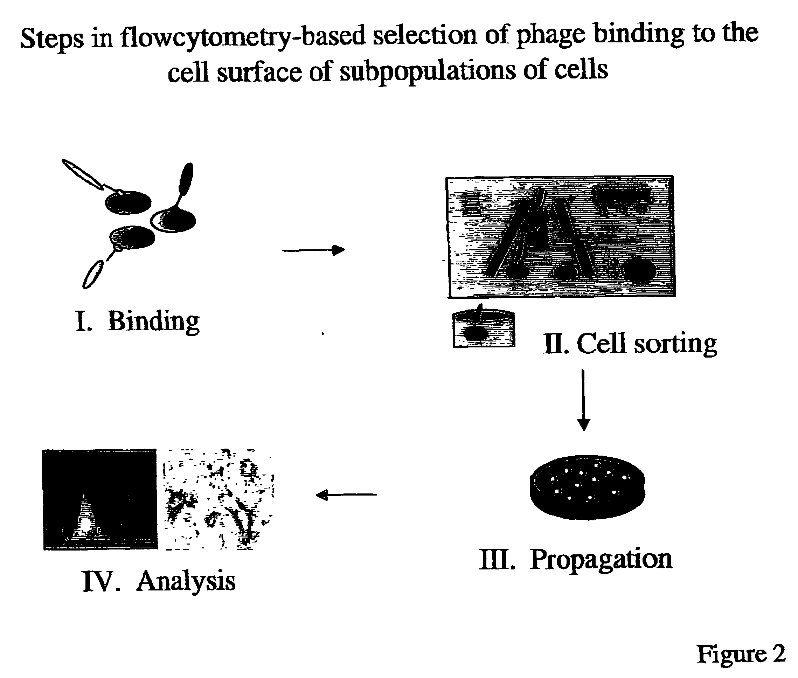

In one particular selection procedure (depicted in Fig.2), phage display libraries are used in combination with flow cytometry and cell sorting to isolate antibody fragments against molecules expressed on the plasma membrane of subpopulations of eukaryotic cells present in a heterogeneous mixture (De Kruif et al. 1995a). Thus, the heterogeneous mixture of cells is incubated with the phage library allowing phages to bind to the different cell types. Subsequently, the cells are stained with fluorochrome-labelled monoclonal antibodies to permit identification of the subpopulation of target cells by immunofluorescence analysis and flow cytometry. Target cells and attached phages are collected by flow cytometry and the attached phages are eluted and propagated. This method is rapid, independent of the immunogenicity of the target antigen and yields antibody fragments against molecules in their native configuration. Specific antibodies against very small populations of cells in a heterogeneous mixture can be obtained (De Kruif et al. 1995a and 1996).

For production of intact human monoclonal antibodies, scFv with desirable specificities can be inserted into mammalian expression vectors containing the genes encoding human immunoglobulin constant regions. We have recently developed a series of constructs that permit the rapid conversion of phage display library-derived scFv antibody fragments to fully human monoclonal antibodies of each immunoglobulin isotype and subclass (Huls et al. 1999; Boel et al. 2000). Transfected cell lines harboring these constructs produce human monoclonal antibodies in vitro that are correctly assembled and glycosylated.

It has been proven that treatment with monoclonal antibodies or antibody-derivatives directed against tumor surface antigens is a rational strategy in tumor immunotherapy. This therapy in cancer is aimed at recruiting the humoral and/or cellular arms of the immune system to eradicate tumour cells. To that end, a variety of approaches has been tested in in vivo and in vitro systems including unconjugated antibodies, bi-specific antibodies, immunotoxins, radiolabeled monoclonal antibodies, immunoliposomes and cytotoxic T lymphocytes (reviewed in Renner and Pfreundschuh 1995; Schneider-Godicke and Riethmuller 1995; Vile and Chong 1996; Maloney and Press 1998; Curnow 1997). For example, treatment of a large cohort of patients with resected Dukes C colorectal cancer with a murine monoclonal antibody against the Ep-CAM molecule expressed on colorectal tumor cells has been proven to be very effective. After 7 years of follow-up evaluation, overall mortality of patients was reduced by 32% and the recurrence rate was decreased by 23% (Riethmuller et al. 1998). In another example, a humanized monoclonal antibody against the HER2/neu receptor that is over-expressed on the tumor cells of 25-30% of patients with breast cancer, was shown to slow the progression of tumor growth and to increase the percentage of patients who experienced tumor cell shrinkage (Baselga et al. 1996). In another example, a chimeric monoclonal antibody against the CD20 antigen was shown to give a response in 48% of patients with relapsed low grade or follicular lymphoma (McLaughlin et al. 1998).

In the examples mentioned supra, treatment of patients with the monoclonal antibody is sufficient for clinical effect. Binding of the monoclonal antibody to the target antigen results in the recruitment of components of the complement system and effector cells of the immune system with Fc receptor for antibody constant regions that act in concert to kill the tumor cell. For reasons unknown, binding of a monoclonal antibody to a target antigen on the membrane of a cell does not always result in tumour cell killing. For some antigens it is necessary to arm the monoclonal antibody with entities that kill the tumor cells via other mechanisms such as toxins or radioactive molecules.

In the approaches described supra, the specificity of the monoclonal antibody employed is crucial. Ideally, the monoclonal antibody should specifically bind to tumour cells with minimal cross-reactivity with normal tissues. This criterium is not always met as illustrated by the chimeric anti-CD20 monoclonal antibody. The CD20 antigen is expressed by B cell tumours but also by non-malignant immature and mature B lymphocytes. Despite this cross-reactivity with non-malignant B cells, patients receiving treatment with the chimeric anti-CD20 monoclonal antibody causes few side effects and the clinical success is considerable.

The present invention discloses methods and means to treat Multiple Myeloma (MM), which is a hitherto incurable neoplastic disease of the B-cell lineage, characterized by the presence of multifocal loci of monoclonal plasma cells in the bone marrow (BM). The disease can present itself with homogeneous serum immunoglubulin, osteolytic lesions, anemia, uremia, hypercalcemia, hyperviscosity, amyloidosis, secondary immunodeficiency and renal insufficiency. These clinical symptoms are related to the location, tumor load and the pathophysiology of malignant plasma cells. Conventional chemotherapy, like intermittent melphalan and prednisone, remains an unsatisfactory treatment of multiple myeloma with a low response rate of about 50-60%, few complete remissions (CR<5%) and a median survival of only 30-36 months (Alexanian and Dimopoulos 1994; Boccadoro et al. 1997).

Allogeneic Bone Marrow Transplantation (Allo-BMT) may proof beneficial for myeloma patients when the observed graft versus myeloma effect can be maximally exploited and the problems of occurring graft versus host disease are circumvented.

Encouraging results have been published of trials with high-dose chemo(radio) therapy and autologous stem cell transplantation (Tricot et al. 1995; Cunningham et al. 1994; Harousseau et al. 1995; Bjorkstrand et al. 1995; Attal et al. 1996). Specifically when applied early in the course of the disease in phase II studies dose intensification yielded 30-50% complete remissions. From a French collaborative study it was concluded that the most important prognostic factor in the autologous stem cell transplantation procedure is the achievement of a complete remission during initial chemotherapy (Harousseau et al. 1995). The median survival for patients responding to primary treatment was 54 months versus 30 month for the non-responders. Induction of CR is presumably the first step towards cure in MM. Nevertheless, patients in CR after autologous stem cell transplantation show a high relapse rate and there is no plateau in survival curves and therefore it seems unlikely that cure may be achieved by intensive therapy alone. Additional therapeutical strategies should thus be directed at eliminating residual tumour cells.

The present invention provides methods for selecting myeloma specific antigens and their specific interacting proteins. More in particular, the invention discloses binding molecules that selectively interact with tumor specific antigens, whereas the antigens are expressed on myeloma cells, other tumor cells and not on normal CD46 positive cell types. Antibody mediated therapies in myeloma have thus far been rather unsuccessful, partly because of absence of antigens with a plasma cell restricted expression pattern. However recently, vaccination experiments in a mouse model system using a tumour idiotype with comparable patho-biological features as human multiple myeloma, suggest that the immune system can effectively be mobilized against myeloma tumour cells (King et al. 1998).

The invention further provides a novel MM tumor marker namely human CD46, which is also known as Membrane Cofactor Protein (MCP) and it provides more in particular, binding molecules that specifically interact with differentially glycosylated forms of human CD46. CD46 is a one- or two-band profile type 1 membrane protein of approximately 60.000 Dalton in molecular weight and is expressed on all nucleated cells. A soluble form of said protein was also found (Hara et al. 1992). Said protein protects host cells from autologous complement attack and serves as a measles virus receptor, facilitating virus to cell and cell-to-cell attachment and fusion (Nanicke et al. 1993; Dorig et al. 1993; Iwata et al. 1995). The fysiological role of CD46 is to protect the host cell from complement-mediated cell damage (Oglesby et al. 1992; Seya et al. 1990b). CD46 consists of four short consensus repeat domains, known as complement control protein (or CCP) repeats: a Ser/Thr-rich (ST) domain, a 13 amino acid unique sequence followed by a transmembrane (TM) portion and a cytoplasmic (CY) tail. Alternative splicing of CD46 transcripts causes different combinations of the exons of the gene encoding CD46, yielding a number of different isoforms (Liszewski et al. 1991). The amino terminus, identical for all isoforms, contains three potential N-glycosylation sites in CCP1, CCP2 and CCP4. The ST domain is extensively O-glycosylated. The glycosylation of CD46 was suggested to play a role in complement regulatory functions (Liszewski et al. 1998). To date six isoforms of CD46 have been identified in human cell lines, testis and placental cDNA libraries (Post et al. 1991).

Many tumor cells and cell lines express particularly high levels of complement regulatory proteins, like CD46, decay-accelerating factor (DAF, CD55) and protectin (CD59) (Seya et al. 1990a). It has been shown that human CD46 rather than CD55 is a key element in protection against complement activation (Van Dixhoorn, 2000). Overexpression of CD46 was observed in gastro-intestinal tumors, carcinomas of breast, cervix and endometrium, and hepatocellular carcinomas (Murray et al. 2000; Kinugasa et al. 1999; Schmitt et al. 1999; Thorsteinsson et al. 1998; Simpson et al. 1997). The difference in expression profiles of this membrane complement regulatory protein between normal and pathological tissues suggest resistance of tumor tissue to complement-mediated damage, thereby allowing tumor cells to escape from cytolysis and thus promoting tumor outgrowth.

One example of a successful tumor immuno-therapeutical approach is based on antibody and complement regulatory proteins. Overexpression of complement regulatory proteins that inhibit complement dependent cytotoxicity can result in a failure of monoclonal antibody therapy. This phenomenon is demonstrated in vitro by neutralization experiments using specific antibodies directed against complement regulator proteins. (Juhl et al. 1997; Jurianz et al. 1999). These studies using breast carcinoma cells in combination with recombinant monoclonal antibody anti-HER2 or gastrointestinal cancer cells in combination with 17-1A anti-EPCAM revealed that the killing of tumor cells could be significantly increased by the use of antibodies directed against complement regulatory proteins, like CD46. So, anti-CD46 monoclonal antibodies can be very useful to overcome the limitation of the potential of monoclonal antibodies mediated by complement resistance.

The invention provides methods for determining differentially expressed folded and/or post-translationally modified proteins on cells, in particular differentially glycosylated CD46 forms on tumor cells. The potential for structural diversity of glycans in metazoan cells is very large given the possible combinations of monosaccharides, linkages, branching, and variable lengths of glycan chains. The structural variability of glycans is dictated, among others, by tissue specific regulation of glycosyltransferase genes, acceptor and nucleotide availability in the Golgi, compartimentalization, and by competition between enzymes for acceptor intermediates during glycan elongation (reviewed in Dennis et al. 1999). At any particular glycosylation site of a mature glycoprotein, a range of biosynthetically related glycan structures may be present. The prevalence of particular glycan structures on specific glycoprotein molecules can affect their functions, including half-life, localisation and biological activity. Aberrant glycosylation has been associated with tumor cells of epithelial origin. In fact, epitopes expressed by the MUC-I antigen expressed on epithelial tumors are targets for various forms of immunotherapy. Previous experiments have suggested that deveating glycosylation of membrane and secreted proteins may be a characteristic of plasma cells and the malignant cells in multiple myeloma. An incompletely sialylated form of CD44 on a myeloma cell has been described and immunoglobulins produced by myeloma cells have a distinct oligosaccharide profile (Slupsky et al. 1993; Farooq et al. 1997). Proteoglycans of B lymphocytes undergo structural changes during B cell ontogeny which may correspond to the specific requirements of the respective microenvironment of the maturing cell (Engelmann et al. 1995).

The invention demonstrates that tumor cells, for example but not limited to Multiple Myeloma cells, express certain types of glycosylated forms of CD46 proteins, that are not expressed on non-tumor cell types. The invention makes use of this feature and provides also a number of binding molecules and more in particular fully human monoclonal antibodies that specifically interact with the differentially glycosylated human CD46 proteins. In a particularly preferred embodiment of the invention these human monoclonal antibodies are used to treat different kinds of human malignancies, in particular Multiple Myeloma.

### Brief description of the drawings and tables

Figure 1.
   Various forms of recombinant monoclonal antibodies based on murine antibodies (Chimeric and Humanized) and fully human monoclonal antibodies derived from transgenic mice or phage display libraries (Human).
Figure 2.
   Steps in selection of phages binding to subpopulations of cells using flow cytometry. I. A heterogeneous mixture of cells is incubated with the phage library. Non-binding phages are removed by washing. II. Cells with bound phages are stained with fluorochrome-labeled monoclonal antibodies and cells of interest are isolated using a cell sorter. III. Phages are eluted from the isolated cells and used to infect bacteria. Phage antibodies are isolated from single bacteria. IV. Phage antibodies are finally used in flow cytometric and immunohistochemical analysis to assess the tissue and cellular distribution of the target antigen.
Figure 3
   Sort critera for malignant plasma cells from bone marrow mononuclear cells from patients with multiple myeloma based on high levels of CD38 expression and forward scatter/side scatter profile (Terstappen et al. 1990).
Figure 4
   Staining of cell suspensions prepared from blood, spleen, tonsil and adult bone marrow (ABM) of healthy individuals and bone marrow of patients with multiple myeloma (MMBM) with a phage directed to thyroglobuline (control) or with K53.
Figure 5
   Amino acid sequence of scFv antibodies K19, K29 and K53 and VH and VL gene utilization (* Nomenclature according to Matsuda and Honjo (1996)).
Figure 6
   Staining of cell suspension prepared from bone marrow of patients with multiple myeloma with K53 and positive cells were isolated by cell sorting and stained with May Grunwald Giemsa
Figure 7
   Expression cloning of K19 binding molecule using a human placental cDNA library cloned in baculovirus.
Figure 8
   Binding to glycosylation variants of CD46 by human monoclonal antibodies K19, K53 and the conventional murine anti-CD46 antibody. Binding was determined in CHO cells, which were stably transfected with normal full length CD46 (BC1), with NQ replacements of CCP1 (NQ1)/ CCP2 (NQ2)/ CCP4 (NQ4) or with a CD46 deletion mutant in the serine/threonine/proline rich domain (ΔSTP).
Figure 9
   FACS analysis showing the binding of anti-CD46 monoclonal antibody K53/IgG1 and the negative control antibody GBS III/IgG1 to bone marrow cells isolated from myeloma patients costained with CD38 and CD138.
Figure 10
   Mean tumor size in NOD/SCID mice xenografted with colon carcinoma cell line LS174T and treated with K53/IgG1 or control GBS III/IgG1 human monoclonal antibodies.

**Table I**

| Bindingcapacity of K19, K29, K53 and tyroglobuline (control) to different tumor cell lines analysed by FACS. | | | | |
|---|---|---|---|---|
| cell line | control | K19 | K29 | K53 |
| HeLa | 4 | 33 | 62 | 55 |
| HepG2 | 5 | 55 | 105 | 93 |
| MCF-7 | 6 | 89 | 128 | 157 |
| LS174T | 6 | 48 | 56 | 125 |
| COS-7 | 8 | 10 | 11 | 7 |

**Table II**

| Complement-dependent cytotoxicity (CDC), antibody-dependent cellular cytotoxicity (ADCC) and complement plus antibody-dependent cellular cytotoxicity (CDCC) using human K53/IgG1, K29/IgG1 and GBS III/IgG1 human monoclonal antibodies (20 µg/ml). The numbers are expressed as percentage of cytotoxic LS174T colon cancer cells and are the mean of 8 different donors. | | | | |
|---|---|---|---|---|
| huMabs | control | CDC | ADCC | CDCC |
| - | 9.9 | 7.7 | 10.1 | 8.9 |
| GBSIII/IgG1 | 8.5 | 6.2 | 11.6 | 13.2 |
| K53/IgG1 | 10.1 | 4.9 | 37.2 | 33.7 |
| K29/IgG1 | 9.9 | 4.5 | 20.7 | 18.9 |

### Examples

### Example 1: Isolation of plasma cell-specific phage antibodies K19, K29 and K53 for a semi-synthetic phage antibody display library.

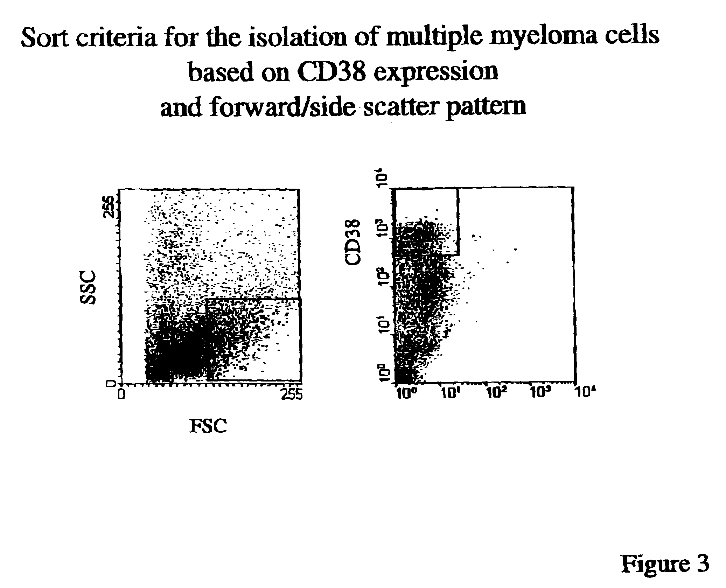

Phage antibody display in combination with flow cytometry (Fig.2) was used to isolate human scFv antibody fragments that bind to malignant plasma cells. Details of this method have been described elsewhere (De Kruif et al. 1995a and 1995b). The preparation of cell suspensions from blood, tonsil, spleen and bone marrow from healthy individuals and multiple myeloma patients is described in Van der Vuurst de Vries and Logtenberg (2000). Mononuclear cells from these suspensions were isolated by Ficoll-Paque (Pharmacia) density centrifugation and subsequent washes in PBS plus 1% Bovine Serum Albumine and used for FACS analysis as described (Van der Vuurst de Vries and Logtenberg 2000). Bone marrow mononuclear cells of a patient with Multiple Myeloma were mixed with a phage display library of human scFv fragments.
Malignant plasma cells were isolated by flow cytometry based on high levels of CD38 expression and forward/side scatter profile (Fig.3). Phages bound to the sorted malignant plasma cells were eluted and propagated by infection of XL1-blue bacteria as described in detail elsewhere (De Kruif et al. 1995b). After three rounds of selection on the malignant plasma cells of three different patients, phages were prepared from individual bacterial colonies and used in immuno-fluorescent analysis as described (De Kruif et al. 1995a). Phage antibodies that bound to malignant plasma cells in the bone marrow of patients with Multiple Myeloma and that showed little staining (as performed by De Kruif et al. 1995a) of hematopoietic cells in other lymphoid organs were selected for further study. Three specifically and strongly interacting phage antibodies were identified and named K19, K29 and K53.

### Example 2: Specific interaction of K19, K29 and K53 antibodies with Multiple Myeloma cells.

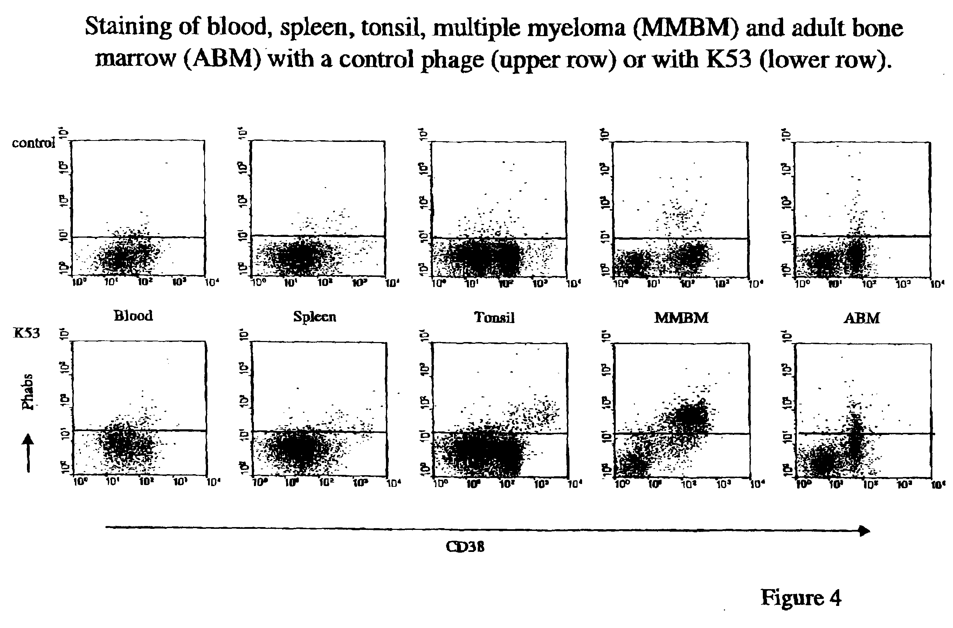

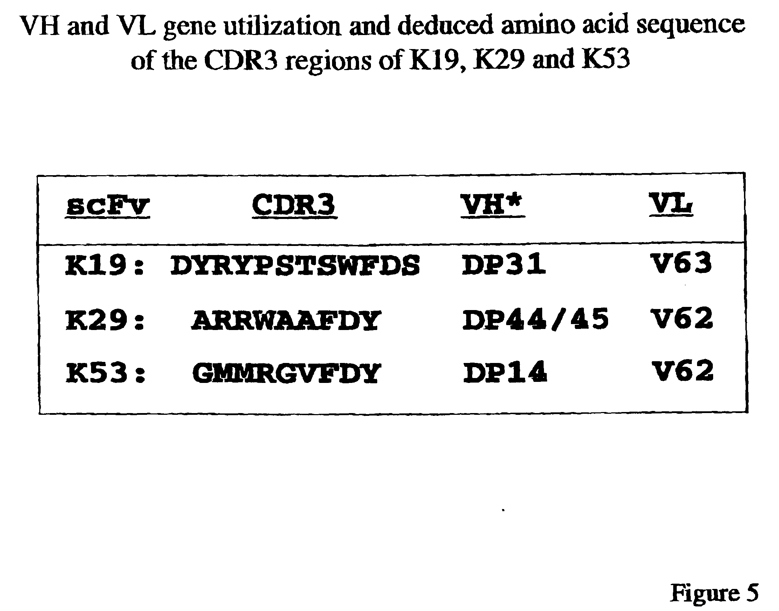

Cell suspensions were prepared from blood, tonsil, bone marrow and spleen of healthy individuals and subsequently stained with the three phage antibodies K19, K29 and K53. For this, cells were stained with myc-tagged single chain Fv fragments followed by anti-myc antibody (9E10) and fluorochrome labeled goat anti-mouse antibodies (Southern Biotechnology Associates). The details concerning the use of 'all these antibodies were described in De Kruif et al. (1995a and 1995b). Subsequently, binding capacity was analyzed by FACS. These antibodies only bound to the malignant plasma cells in the bone marrow of patients with multiple myeloma and to a small population of CD38^{bright} cells in tonsil and normal bone marrow. No staining of hematopoietic cells in spleen or blood was observed. Fig.4 shows the binding of K53 to these cells as compared to a control phage antibody which is directed against thyroglobuline. The nucleotide sequence of the three scFv antibodies was determined and unveiled that K19, K29 and K53 were encoded by different immunoglobulin heavy and light chain variable genes (Fig.5) and thus represented three independent scFv antibody fragments. The FDY motif that seems to overlap between these antibodies is present in many (also non-CD46 binding) antibodies. The determination of crucial residues involved in the CD46 specific interaction is investigated and described in more detail in example 11.

### Example 3: Staining of bone marrow cells from a Multiple Myeloma patient by K53.

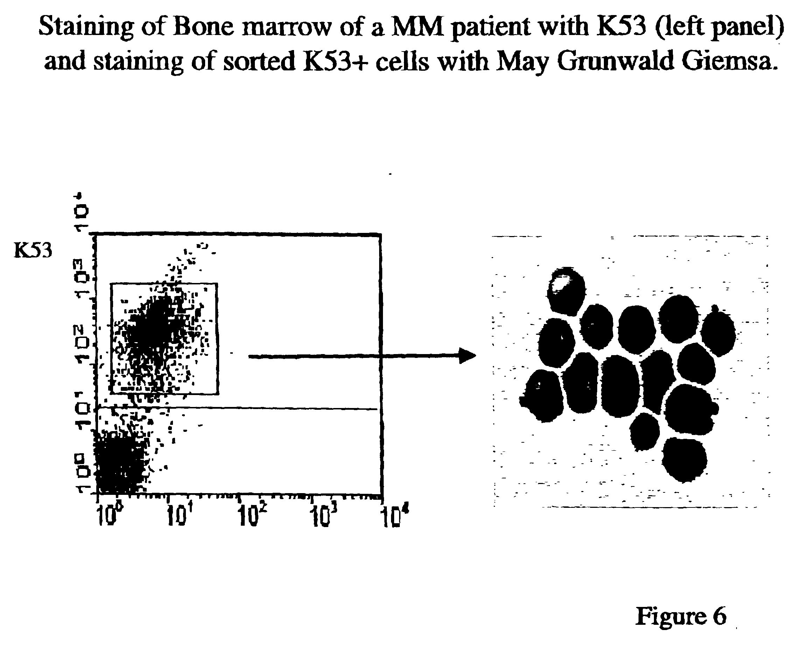

CD38^{bright} cells are cells that exhibit a very high CD38 expression (black dots in Fig.3). Cells of this kind are found in bone marrow and tonsil and have been previously shown to represent plasma cells and precursors of plasma cells, or so-called plasma blasts (Terstappen et al. 1990). In order to confirm that the scFv antibody fragments recognized plasma cells, bone marrow cells from a MM patient were stained with scFv K53 and positive cells were isolated by cell sorting. For this, isolation, staining and cell sorting procedures were performed as described by De Kruif et al. (1995a) and Van der Vuurst de Vries and Logtenberg (2000). Cytospin preparations (80,000 cells were spun in 100 µl PBS at 500 rpm during 5 min) from the sorted cells were stained with May Grunwald Giemsa (12% solution during 15 min in aquadest) en cells were visualized by light microscopy. As shown in Fig.6, sorted K53+ bone marrow cells displayed a distinct plasma cell morphology.

### Example 4: Determination of the human CD46 protein as the antigen that is specifically recognized by the MM specific scFv antibody fragments.

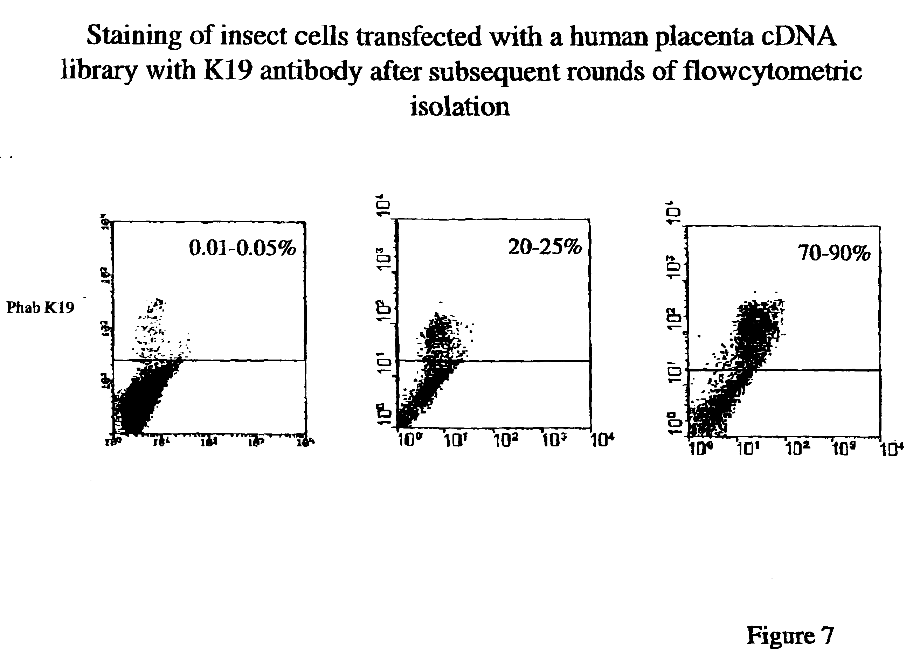

The phage antibody K29 was used for expression cloning using a human placental cDNA library cloned in baculovirus. By staining with phage antibodies, single positive cells can be sorted. Subsequently the virus, which encodes the surface epitope can be isolated by limiting dilution. Details of the cDNA library, the use of the library and virus identification by limiting dilutions are described by Granziero et al. (1997). The results of these experiments are shown in Fig.7. Nucleotide sequence analysis was subsequently performed by methods known to persons skilled in the art. The analysis of the cDNA inserts from clones obtained from two size-selected placental cDNA libraries, containing inserts smaller than 2 kb and inserts larger than 2 kb respectively, unveiled open reading frames that in both instances completely matched the reported nucleotide sequence of the human CD46 gene. Re-introduction of the human CD46 cDNA clones in Sf9 insect cells was performed using Lipofectamine transfection procedures (Life Technologies) and staining with scFv antibody fragments K19, K29 and K53 confirmed that all three scFv antibodies recognized the CD46 gene.

### Example 5: Recognition of CD46 subforms by the Multiple Myeloma specific scFv antibodies.

There is an apparent discrepancy between the broad expression of the CD46 gene (as detected with conventional murine anti-CD46 antibodies) and the plasma cell-restricted expression of the CD46 epitopes (as detected by the phage display library-derived scFv antibody fragments). This was further investigated by using a panel of glycosylation mutants of the CD46 molecule that were stably expressed in Chinese Hamster Ovary (CHO) cells. In these CD46 mutants, an asparagine in CCP-1, CCP-2 or CCP-4 was replaced by a glutamine resulting in disruption of the N-glycosylation site. In a fourth construct, the entire STP domain with its O-Linked glycosylation sites was deleted. All constructs, the generation of stable cell lines and the culturing of these cells were described by Liszewski et al. (1998). Binding of the antibodies to the CD46 glycosylation mutants expressed on the cell surface of the stable cell lines were monitored by immunofluorescent analysis in combination with an anti myc-tag (9E10) antibody and PE linked goat-anti mouse secondary antibody (Southern Biotechnology Associates).

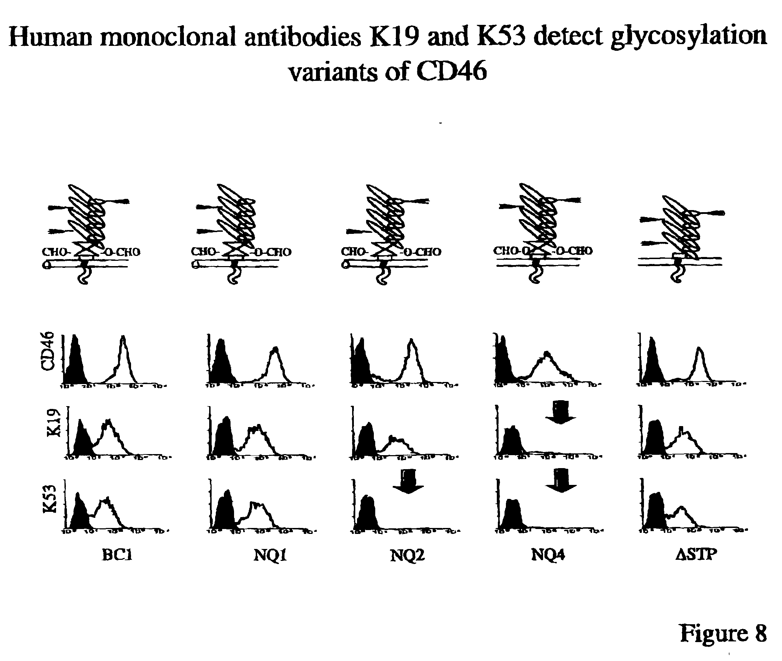

In immunofluorescent analysis, the murine anti-CD46 antibody (J4.48 Immunotech) stained CHO cells transfected with the wildtype CD46 cDNA as well as the transfectants in which each of the glycosylation sites in the CCP domains or the entire STP domain (ΔSTP) were deleted (Fig.8). In contrast, different staining patterns were obtained with the scFv K19, K29 and K53 antibody fragments. The staining pattern of K19 and K29 were comparable. All three antibodies specifically stained the cell line transfected with the wildtype CD46 cDNA, as well as the CCP-1 and STP glycosylation mutants (Fig.8). K19 and K29 also bound to the CCP-2 glycosylation mutant but completely failed to bind to the CCP-4 glycosylation mutant. K53 lost binding to the CCP-2 and CCP-4 glycosylation mutants. Thus, the epitopes on CD46 recognized by K19, K29 and K53 are all dependent on the N-glycosylation of CD46. The involvement of N-glycosylation 'in binding to CD46 by the huMabs can also be demonstrated by using the glycosylation inhibitor tunicamycin (see also example 7).

### Example 6: Specific binding of CD46 monoclonal antibodies to myeloma and leukemic tumors.

Bone marrow cells from healthy individuals and from MM patients were screened for K53/IgG1 huMab binding. For this, the scFv fragments were first converted into intact, fully human IgG1 monoclonal antibodies by recloning the heavy and light chain variable regions in eukaryotic expression vectors (Boel et al. 2000) and by co-expressing the heavy and light chain constructs in Baby Hamster Kidney (BHK) cells (Huls et al. 1999).

The derived K53/IgG1 and control GBS III huMabs were labeled with PE or APC (performed by IQ Systems, The Netherlands). The bone marrow cells were incubated with the huMabs in the presence of 10% normal human serum (NHS) during 15 min at room temperature and were analyzed by FACS. To characterize the positive cells, the tissues were co-stained with several (labeled) mouse monoclonal antibodies against the myeloma markers CD38 and CD138, and against CD45, CD19 and CD3.

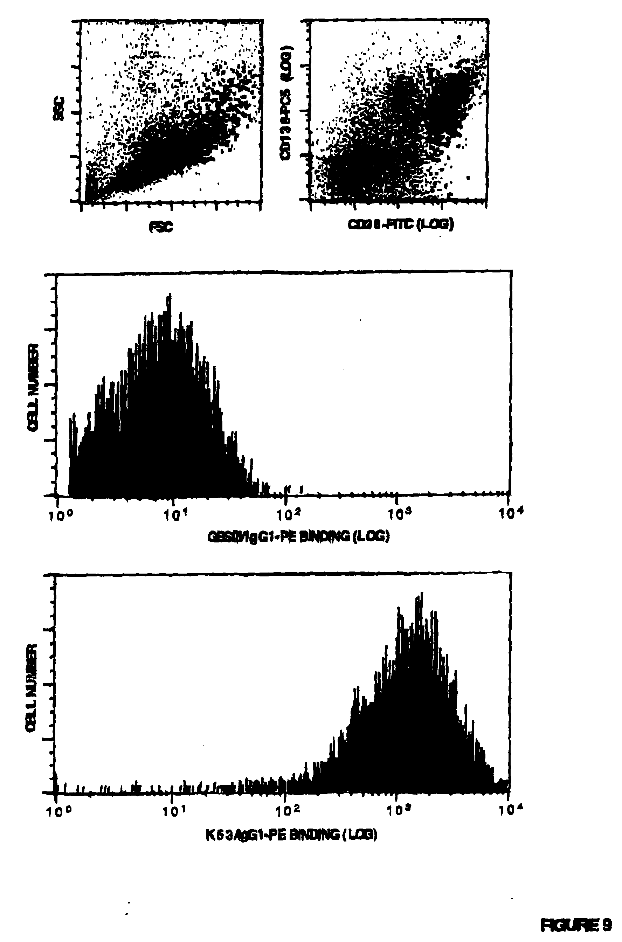

The K53/IgG1 monoclonal antibody co-stained with the myeloma markers CD38 and CD138 (Fig.9). Binding of K53/IgG1 monoclonal antibody was intact when material was frozen with liquid nitrogen. In contrast, binding of the mouse monoclonal antibody directed against the myeloma marker CD138 with Multiple Myeloma cells is disrupted after freezing with liquid nitrogen, which limits the use of this monoclonal antibody. Therefore, the combination of anti-CD38 monoclonal antibody with the K53/IgG1 is very useful as a diagnostic marker. Binding was not detected in bone marrow cells from healthy individuals and when the control human GBS III antibody was used. Also FACS experiments with whole blood of healthy persons was negative for all the human antibodies used. This negative result was also found with other types of leukemic tumors like Chronic Lymphatic Leukemia (CLL). So, using normal leukocytes and cells from leukemic tumors, the K53/IgG1 clearly binds specifically to Multiple Myeloma cells.

### Example 7: Binding of anti-human CD46 monoclonal antibodies to solid tumors and cells derived from solid tumors.

One of the functions of the CD46 protein is to protect the cell from autologous complement attack. One mechanism for tumor cells to deviate the complement attack is by overexpressing the CD46 protein. This over-expression is found in several neoplasia like breast-, cervix-, liver-, colorectal- and gastro-intestinal cancer. The results shown in example 5 suggest that CD46 can not only be over-expressed, but also differentially glycosylated. The MM-specific K19, K29 and K53 antibodies apparently distinguish between these different glycosylation forms (Fig.8). To investigate whether the K19, K29 and K53 antibodies also bind to tumor cells that over-express CD46, said antibodies were incubated with a set of tumor cell lines (MCF-7 (breast), HeLa (cervix), HepG2 (liver) and LS174T (colon)) and determined the staining with fluorescent labeled antibodies as described supra. Table I shows the increase in interaction between several solid tumor derived cell lines with K19, K29 and K53 antibodies. Taken together with the data described in example 5, these data suggest that these cells not only exhibit an over expression of the CD46 protein but that the protein also has a different glycosylation pattern as compared to wild type, non-tumor cells.

It is concluded that the antibodies interact with CD46 proteins on cells that were derived from solid tumors. It is also tested whether said antibodies also interact with CD46 present on cells in solid tumors and not with surrounding tissues. Therefore, tumor material is obtained directly after surgery or pathology and stained with the different anti-human CD46 antibodies.

To determine the role of N-glycosylation in K53/IgG1 binding, K53/IgG1 positive tumor cell lines (LS174T cells and MCF7 cells) are treated with the N-glycosylation inhibitor tunicamycin. The cells are stained for CD46 huMabs and commercial murine anti-CD46 antibody (J4.48), which binds to all CD46 derivates. For this purpose, PE-linked huMabs and the murine anti-CD46 antibody are used in combination with a second PE-labeled antibody. The binding capacity is detected and analyzed by Fluorescence-Activated Cell Sorting (FACS).

### Example 8: ADCC and CDCC assays

The tumor cell killing activity of the anti-CD46 antibody using human peripheral blood mononuclear cells was evaluated using Multiple Myeloma cells and tumor cell line LS174T derived from colon as target cells in Antibody and Complement Dependent Cellular Cytotoxicity (ADCC and CDCC) assays. The target cells were labeled with 30 ng/ml calcein for 5 min at 37°C. After extensive washing, isolated human mononuclear cells were added to the target cells at an effector to target ratio of 40:1. Complement mediated lysis was performed with 50 µl serum in a final volume of 200 µl. Cells were incubated at 37°C in the presence of various concentrations of CD46 human antibodies, Group B Streptococcus antigen III specific (GBS III) control antibodies or PBS. After 4 hours, propidium iodide was added as a marker for cytotoxicity and cytolysis was determined by FACS analysis. The percentage cellular cytotoxicity was 'calculated as follows: % specific cytolysis = [number of double positive (propidium-iodide positive and calcein positive) cells/ total number of calcain positive cells] x 100. Cytotoxicity was determined in the presence of serum (CDC), in the presence of effector cells (ADCC) and in the presence of both (CDCC). The results of K53/IgG1 and K29/IgG1 are shown in Table II and clearly indicate that both human monoclonal antibodies directed against CD46 glycoforms are effective in killing target cells in the presence of blood effector cells.

### Example 9: In vivo tumor cell killing by anti-CD46 in a xenograft model of colon carcinoma.

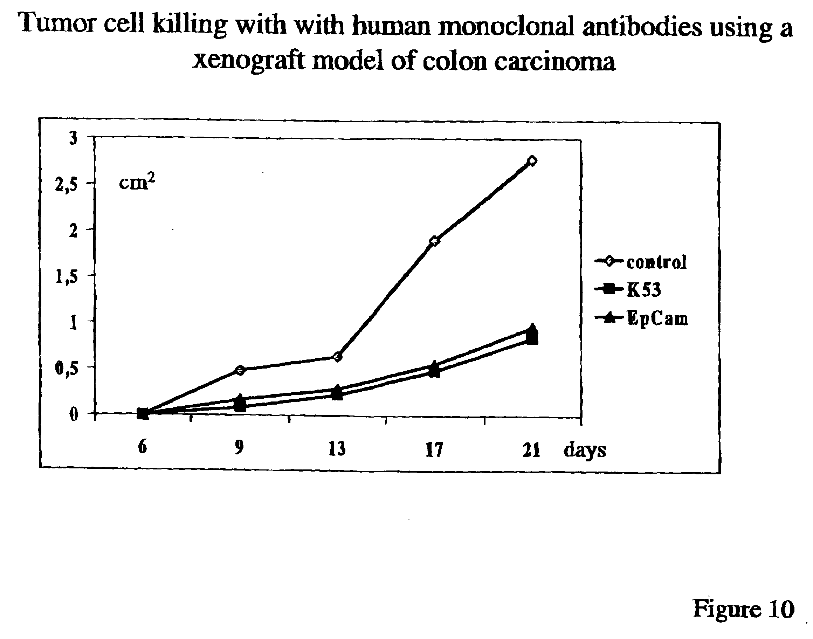

To evaluate the in vivo tumor cell killing capacity of the anti-CD46 monoclonal antibodies, nine-week old NOD/SCID mice were injected subcutaneously into both flanks with one million LS174T human colon carcinoma cells (day 0). The next day, two groups of 6 animals each were treated: one group with 200 µg K53/IgG1 and the other (control) group with 200 µ g GBS III/IgG1 human monoclonal antibodies. On day 3 and 6, the treatment was repeated with 100 µg monoclonal antibodies. The treatment effects were evaluated by measuring the mean tumor size (maximal length x maximal width) during 3 weeks using methods known to people skilled in the art. Significantly, the tumor growth was markedly retarded by the CD46 human monoclonal antibody (Fig.10).

### Example 10: Determination of oligosaccharide content of CD46 present on tumor cells that are specifically recognized by K19, K29 and K53.

The glycosylation status of the CD46 protein present on normal and cancer related cells, recognized by K19, K29, K53 and other binding molecules is determined. Amongst others, CD46-positive tumor cells (MCF-7, LS174T and MM) and CD46-negative Peripheral Blood Lymphocytes (PBL) are subjected to immunoprecipitation using different CD46 specific monoclonal antibodies and methods known to persons skilled in the art. This is followed by western blot analysis using HRP-linked lectins. These lectins discriminate between different sugar molecules present in the oligosaccharide backbones and are used for western blotting according to methods described by the manufacturers. The lectins can specifically recognize the following molecules: A. High mannose (con A from Canavalia ensiformis), B. Higher branched complex type oligosaccharides (WGA from Triticum vulgare and PHA-L from Phaseolus vulgaris), C. Sialic acids (LFA from Limax flavus) and D. Fucose residue (UEX-I from Ulex europaeus). The glycosylation status of the CD46 protein is determined on sets of tumor cells and compared to the status of the oligosaccharides present on CD46 which is expressed on the surface of normal cells or which is soluble and present in bodily fluids in healthy individuals and cancer patients. Moreover, CD46 glycosylation is detected also within one tumor type and/or one tumor derived cell line.

These experiments are followed by procedures to find which type(s) of glycosylation is responsible for binding to which binding molecule, more in particular to the antibodies K19, K29 and K53 and what glycosylation patterns and sugar backbones are overlapping. These procedures include polysaccharide analysis with NMR and mass spectrometry using procedures well known in the art. Subsequently, after determining what glycoforms of the CD46 protein are present on what tumor cells, the correct monoclonal antibody will be used to induce complement activation by the best interacting binding molecule in in vitro and in in vivo studies.

### Example 11: Specification of amino acid residues in the variable domain of the antibodies that determine the binding to different CD46 glycoforms.

The residues in the variable domains in the antibodies that are selected using the methods described supra are responsible for the specific interaction between the CD46 protein and the recombinant IgG1. Moreover, they specify the interaction with the different glycosylation patterns that are present on said protein expressed on the surface of Multiple Myeloma cells and other cells derived from solid tumors. The responsible amino acid residues are determined by randomly altering the amino acid order present in CDR3 and other variable regions. Subsequently, the mutagenized regions are incorporated into full IgG1 molecules, expressed, purified and used in binding assays with MM and other tumor derived cells. Positive binding molecules are subsequently selected.

### Example 12: Production of recombinant fully human monoclonal antibodies K19/IgG1, K29/IgG1 and K53/IgG1 in human PER.C6 cells.

It is possible that recombinant monoclonal antibodies that are selected by the methods described supra, do render immune responses in humans that are being treated with these binding molecules due to the fact that there might be unrelated non-human post translational modifications present on these therapeutic molecules. For this it is preferred to produce these fully human monoclonal antibodies in a system that comes closest to the human situation. Therefore, heavy and light chains of the fully human monoclonal antibodies K19/IgG1, K29/IgG1 and K53/IgG1 are cloned in several eukaryotic expression vectors that are described, amongst others, by Huls et al. (1999). These vectors are subsequently transfected into human PER.C6 cells using transfection procedures well known to persons skilled in the art. Then, cells that have stably integrated versions of both heavy and light chain are selected by double selection with G418 (Life Techn.) and Hygromycin (Life Techn.). Another procedure involves subsequent selection in which first Hygromycin is added to the medium in which the cells are growing and outgrowing colonies are further selected with the addition of G418 to the medium. In other systems, both heavy and light chain are under the same selection pressure because they are cloned in one expression vector or they are cloned in similar expression vectors carrying the same selection marker. In yet another method, a Methotrexate-DHFR slection method (Urlaub et al. 1983) is applied in which it is possible to amplify the integrated plasmids and thereby increasing the production levels of the recombinant antibodies. Positive cell clones are picked and subcultured according to methods known to persons skilled in the art. Then specific production rates are determined and the best clones are selected for further outgrowth, banking, stability of expression, amplification, suspension growth and optimal growth versus production in large bioreactors. The recombinant fully human monoclonal antibodies directed against the differentially glycosylated CD46 proteins are purified from the supernatant using methods well known to persons skilled in the art and used in specificity experiments, ADCC and CDCC assays and in vivo tumor-killing experiments. These fully human monoclonal antibodies produced in human cells are furthermore used in specificity- and pharmacokinetics studies and half-life experiments.

### Example 13: Neutralization of CD46 augments killing of tumor cells targeted with UBIS 54 anti-Ep-CAM.

To detect the effect of neutralizing complement regulatory proteins like CD46, the following experiments are performed. A million CD46 overexpressing tumor cells (LS174T) are injected subcutaneously in immune deficient mice and treated one day later with 100 µg human anti-Ep-CAM (UBIS 54) monoclonal antibody and/or 100 µg human K53/IgG1 monoclonal antibody. On day 3 and 6, the treatment is repeated with 50 µg UBIS 54 monoclonal antibody and/or 50 µg human K53/IgG1 monoclonal antibody. As a control a Streptococcus specific GBS III antibody is used (see above). The effect of monoclonal antibody treatment is evaluated by measuring the mean tumor size (maximal length x maximal width) during 3 weeks (see above).

### References

Alexanian R, Dimopoulos M (1994) The treatment of multiple myeloma. N Engl J Med 7:484
Attal M, Harousseau JL, Stoppa AM, et al. (1996) A prospective, randomized trial of autologous bone marrow transplantation and chemotherapy in multiple myeloma. N Engl J Med 335:91
Baselga J, Tripathy D, Mendelsohn J, et al. (1996) Phase II study of weekly intravenous recombinant humanized anti-p185HER2 monoclonal antibody in patients with HER2/neu overexpressing metastatic breast cancer. J Clin Oncol 14:737
Bjorkstrand B, Ljungman P, Bird JM, et al. (1995) Autologous stem cell transplantation in multiple myeloma: results of the European Group for Bone Marrow Transplantation. Stem Cells 13:140S
Boccadoro MA, Alexanian R, Barlogie B (1997) Diagnosis, prognosis and standard treatment of multiple myeloma. Clin Hematol Oncol North Am 11:111
Bodey B, Bodey Jr B and Siegel SE (2000) Genetically-engineered monoclonal antibodies for direct anti-neoplastic treatment and cancer cell-specific delivery of chemotherapeutic agents. Curr Pharm Des 27:49
Boel E, Verlaan S, Poppelier MJJG, et al. (2000) Functional human monoclonal antibodies of all isotypes constructed from phage display library-derived single chain Fv antibody fragments. J Immunol Methods 239:153
Bruggeman M and Neuberger MS (1996) Strategies for expressing human antibody repertoires in transgenic mice. Immunol Today 17:391
Burton DR and Barbas III CF (1994) Human antibodies from combinatorial libraries. Adv Immunol 57:191
Cunningham D, Paz-Ares L, Milan S, et al. (1994) High-dose melphalan and autologous bone marrow transplantation as consolidation in previously untreated myeloma. J Clin Oncol 12:759
Curnow RT (1997) Clinical experience with CD64-directed immunotherapy. An overview. Cancer Immunol Immunother 45:210
De Kruif J, Terstappen L, Boel E et al. (1995a) Rapid selection of cell subpopulation-specific human monoclonal antibodies from a synthetic phage antibody library. Proc Natl Acad Sci USA 92:3938
De Kruif J, Boel E, Logtenberg T (1995b) Selection and application of human single chain Fv antibody fragments from a semi-synthetic phage antibody display library with designed CDR3 regions. J Mol Biol 248:97
De Kruif J, Van der Vuurst de Vries A-R, Cilenti L, et al. (1996) New perspectives on recombinant human antibodies. Immunol Today 17:453
Dennis JW, Granowsky M and Warren CE (1999) Protein glycosylation in development and disease. BioEssays 21:412
Dorig RE, Marcil A, Chopra A, et al. (1993) The human CD46 molecule is a receptor for measles virus (Edmonston strain). Cell 75:295
Elliot MJ, Maini RN, Feldman M, et al. (1994) Repeated therapy with monoclonal antibody to tumor necrosis factor alfa (cA2) in patients with rheumatoid arhritis. Lancet 344:1125
Engelmann S, Ebeling O, Schwartz-Albiez R (1995) Modulated glycosylation of proteoglycans during differentiation of human B lymphocytes. Biochim Biophys Acta 1267:6
Farooq M, Takahashi N, Arrol H, et al. (1997) Glycosylation of polyclonal and paraprotein IgG in multiple myeloma. Glycoconj J 14:489
Foote J and Winter G (1992) Antibody framework residues affecting the the conformation of the hypervariable loops. J Mol Biol 224:487
Granziero L, Nelboeck P, Bedoucha M, et al. (1997) Baculovirus cDNA libraries for expression cloning of genes encoding cell-surface antigens. J Immunol Methods 203:131
Hakimi J, Chizzonite R, Luke DR, et al. (1991) Reduced immunogenicity and improved pharmacokinetics of humanized anti-Tac in cynomolgus monkeys. J Immunol 147:1352
Hara T, Kuriyama S, Kiyohara H, et al. (1992) Soluble forms of membrane cofactor protein (CD46, MCP) are present in plasma, tears, and seminal fluid in normal subjects. Clin Exp Immunol 89:490
Harousseau J-L, Attal M, Divine M, et al. (1995) Autologous stem cell transplantation after first remission induction treatment in multiple myeloma: a report of the French Registry on Autologous Transplantation in Multiple Myeloma. Blood 85:3077
Huls GA., Heijnen IAFM., Cuomo ME., et al. (1999) A recombinant, fully human monoclonal antibody with antitumor activity constructed from phage-displayed antibody fragments. Nature Biotechnol 17:276
Iwata K, Seya T, Yanagi Y, et al. (1995) Diversity of sites for measles virus binding and for inactivation of complement C3b and C4b on membrane cofactor protein CD46. J Biol Chem 270:15148
Jones PT, Dear PH, Foote J, et al. (1986) Replacing the complementarity-determining regions in a human antibody with those from a mouse. Nature 321:522
Juhl H, Helmig F, Baltzer K, et al. (1997) Frequent expression of complement resistance factors CD46, CD55 and CD59 on gastrointestinal cancer cells limits the theurapeutical potential of monoclonal antibody 17-1A. J Surg Oncol 64:222
Jurianz K, Maslak S, Garcia-Schuler H, et al. (1999) Neutralization of complement regulatory proteins augments lysis of breast carcinoma cells targeted with rhumAb anti-HER2. Immunopharmacology 42:209
Khazaeli MB, Saleh MN, Liu TP, et al. (1989) Pharmacokinetics and immune response of 131I-chimeric mouse/human B72.3 (human gamma4) monoclonal antibody in humans. Cancer Res 51:5461
King CA, Spellerberg MB, Zhu D, et al. (1998) DNA vaccines with single-chain Fv fused to fragment C of tetanus toxin induce protective immunity against lymphoma and myeloma. Nature Med 4:1281
Kinugasa N, Higashi T, Nouso K, et al. (1999) Expression of membrane cofactor protein (MCP, CD46) in human liver diseases. Br J Cancer 80:1820
Köhler G and Milstein C (1975) Continuous cultures of fused cells secreting antibody of predefined specificity. Nature 256:495
Liszewski MK, Post TW, Atkinson JP (1991) Membrane cofactor protein (MCP or CD46): newest member of the regulators of complement activation gene cluster Annu Rev Immunol 9:431
Liszewski MK, Leung MK, Atkinson JP (1998) Membrane cofactor protein: importance of N- and O-glycosylation for complement regulatory function. J Immunol 161:3711
Liu YJ, Arpin C (1997) Germinal center development. Immunol Rev 156:111
Maloney.DG, Press OW (1998) Newer treatments for non-Hodgkin's lymphoma: monocloanl antibodies. Oncology 12:63
Matsuda F and Honjo T (1996) Organization of the human immunoglobulin heavy-chain locus. Adv Immunol 62:1
McLaughlin P, Grillo-Lopez AJ, Link BK, et al. (1998) Rituximab chimeric anti-CD20 monoclonal antibody therapy for relapsed indolent lymphoma: half of patients respond to a four-dose treatment program. J Clin Oncol 16:2825
Mendez MJ, Green LL, Corvalan JRF, et al. (1997) Functional transplant of megabase human immunoglobulin loci recapitulates human antibody response in mice. Nature Genetics 15:146
Miller RA, Oseroff AR, Stratte PT et al. (1983) Monoclonal antibody therapeutic trials in seven patients with T-cell lymphoma. Blood 62:988
Murray KP, Mathure S, Kaul R, et al. (2000) Expression of complement regulatory proteins-CD35, CD46, CD55 and CD59- in benign and malignant endometrial tissue. Gynecol Oncol 76:176
Naniche D, Varior-Krishnan G, Cervoni F, et al. (1993) Human membrane cofactor protein (CD46) acts as a cellular receptor for measles virus. J Virol 67:6025
Oglesby TJ, Allen CJ, Liszewski MK, et al. (1992) Membrane cofactor protein (CD46) protects cells from complement mediated attack by an intrinsic mechanism. J Exp Med 175:1547
Post TW, Liszewski MK, Adams EM, et al. (1991) Membrane cofactor protein of the complement system: alternative splicing of serine/threonine/proline-rich exons and cytoplasmic tails produces multiple isoforms that correlate with protein phenotype. J Exp Med 174:93
Renner C and Pfreundschuh M (1995) Tumor therapy by immune recruitement with bispecifc antibodies. Immunol Rev.145:179
Riechmann L, Clark M, Waldmann H et al. (1988) Reshaping human antibodies for therapy. Nature 332:323
Riethmuller G, Holz E, Schlimok G, et al. (1998) Monoclonal antibody therapy for resected Dukes' C colorectal cancer: seven-year outcome of a multicenter randomized trial. J Clin Oncol 16:1788
Schmitt CA, Schwaeble W, Wittig BM, et al (1999) Expression and regulation by interferon-gamma of the membrane-bound complement regulators CD46 (MCP), CD55 (DAF) and CD59 (protectin) in gastrointestinal tumors. Eur J Cancer 35:117
Schneider-Gadicke E, Riethmuller G (1995) Prevention of manifest metastasis with monoclonal antibodies: A novel approach to immunotherapy of solid tumours. Eur J Cancer 31A:1326
Seya T, Hara T, Matsumoto M, et al. (1990a) Quantitative analysis of membrane cofactor protein (MCP) of complement. High expression of MCP on human leukemia cell lines, which is down-regulated during cell differentiation. J Immunol 145:238
Seya T, Hara T, Matsumoto M, et al. (1990b) Complement mediated tumor cell damage induced by antibodies against membrane cofactor protein (MCP, CD46). J Exp Med 172:1673
Shawler DL, Bartholomew RM, Smith LM et al. (1985) Human immune response to multiple injections of murine monoclonal IgG. J Immunol 135:1530
Simpson KL, Jones A, Norman S, et al. (1997) Expression of the complement regulatory proteins decay accelerating factor (DAF, CD55), membrane cofactor protein (MCP, CD46) and CD59 in the normal human uterine cervix and in premalignant and malignant cervical disease. Am J Pathol 151:1455
Slupsky JR, Duggan-Keen M, Booth LA, et al. (1993) The peanut-agglutinin (PNA)-binding surface components of malignant plasma cells. Br J Haematol 83:567
Stephens S, Emtage S, Vetterlei O, et al. (1995) Comprehensive pharmacokinetics of a humanized antibody and analysis of residual anti-idiotypic responses. Immunology 85:668
Terstappen LW, Johnsen S, Segers-Nolten IM, et al. (1990) Identification and characterization of plasma cells in normal human bone marrow by high-resolution flow cytometry Blood 76:1739.
Thorsteinsson L, O'Dowd GM, Harrington PM, et al. (1998) The complement regulatory proteins CD46 and CD59, but not CD55, are highly expressed by glandular epithelium of human breast and colorectal tumour tissues. APMIS 106: 869
Tricot G, Jagannath S, Vesole D, et al. (1995) Peripheral blood stem cell transplant for multiple myeloma: identification of favourable variables for rapid engraftment in 225 patients. Blood 85:588
Urlaub G, Kas E, Carothers AM, et al. (1983) Deletion of the diploid dihydrofolate reductase locus from cultured mammalian cells. Cell 33:405
Van der Vuurst de Vries A-R and Logtenberg T (2000) Dissecting the human peripheral B cell compartment with phage display derived antibodies. Immunology (in press).
Van Dixhoorn MG, Dekker S, Janssen RW, et al. (2000) Human CD46 rather than CD55 is a key element in protection against complement activation in vitro. Transplant Proc 32:916
Vaughan TJ, Osbourn JK and Tempest PR (1998) Human antibodies by design. Nat Biotechnol 16:535
Vile RG, Chong H (1996) Immunotherapy III: Combinatorial molecular immunotherapy: a synthesis and suggestions - Cancer Metast Rev 15:351
Winter G, Griffiths AD, Hawkins RE et al. (1994) Making antibodies by phage display technology. Annu Rev Immunol. 12:433

## Claims

1. A proteinaceous molecule capable of specifically binding to an epitope present in a subset of CD46 proteins.

2. A proteinaceous molecule according to claim 1, capable of distinguishing a subset of CD46 comprising cells.

3. A proteinaceous molecule according to claim 1 or claim 2, capable of binding to a neoplastic cell.

4. A proteinaceous molecule according to claim 3, wherein said neoplastic cell is derived from a hemopoietic cell, a cervix cell, a colon cell, a kidney cell or a liver cell.

5. A proteinaceous molecule according to claim 3 or claim 4, wherein said neoplastic cell is derived from a B-cell.

6. A proteinaceous molecule according to any one of claims 1-5, capable of binding to a Multiple Myeloma cell.

7. A proteinaceous molecule according to any one of claims 1-5, capable of binding to a subset of human CD46 proteins.

8. A proteinaceous molecule according to anyone of claims 1-7, wherein said molecule is an antibody or a functional part or derivative thereof.

9. A proteinaceous molecule according to claim 8, wherein said antibody is human or humanized.

10. A proteinaceous molecule according to any one of claims 1-9, further comprising a tag.

11. A proteinaceous molecule according to claim 10, wherein said tag comprises a toxin and/or a radioactive substance.

12. A method for the treatment of an individual suffering from or at risk of suffering from a disease, comprising administering to said individual a therapeutically acceptable amount of a proteinaceous molecule according to any one of claims 1-11.

13. A method according to claim 12, wherein said disease is a neoplastic disease.

14. Use of a proteinaceous molecule according to any one of claims 1-11 for the preparation of a medicament.

15. Use according to claim 14, for the treatment of neoplastic disease.

16. A method for typing a cell comprising determining whether said cell is capable of specifically binding a proteinaceous molecule according to any one of claims 1-11.

17. Use of an epitope expressed on a subset of CD46 expressing cells as a marker for neoplastic cells.

18. A nucleic acid encoding a proteinaceous molecule according to any one of claims 1-11, or a part involved in CD46 binding of said molecule.

19. A cell comprising a nucleic acid according to claim 18.

20. A cell according to claim 19, wherein said cell is a primate, rodent, bird or plant cell.

21. A cell according to claim 19 or claim 20, wherein said cell is a human cell.

22. A cell according to claim any one of claims 19-21, further comprising a means for the conditional expression of a nucleic acid of interest.

23. A cell according to claim 22, wherein said means comprises a tetracycline responsive expression system.

24. A cell according to any one of claims 19-23, wherein said cell comprises nucleic acid encoding an early protein of adenovirus or a functional part, derivative and/or analogue thereof.

25. A cell according to claim 24, wherein said early protein comprises adenovirus E1 or a functional part, derivative and/or analogue thereof.

26. A cell according to claim 24 or claim 25, comprising adenovirus E2A or a functional part, derivative and/or analogue thereof.

27. A plant or a non-human animal comprising a cell according to any one of claims 19-26.

28. A non-human animal or plant according to claim 27, transgenic for a nucleic acid according to claim 18.

29. A gene delivery vehicle comprising a proteinaceous molecule according to anyone of claims 1-11.

30. A kit comprising a proteinaceous molecule according to anyone of claims 1-11.
